(19) Europäisches Patentamt
European Patent Office
Office européen des brevets

(11) Veröffentlichungsnummer: **0 118 392**
**B1**

(12) # EUROPÄISCHE PATENTSCHRIFT

(45) Veröffentlichungstag der Patentschrift: **12.08.87**

(51) Int. Cl.⁴: **C 07 D 207/323**, A 01 N 47/34

(21) Anmeldenummer: **84810063.2**

(22) Anmeldetag: **03.02.84**

(54) **Benzoylharnstoff-Verbindungen als Schädlingsbekämpfungsmittel.**

(30) Priorität: **09.02.83 CH 726/83**

(43) Veröffentlichungstag der Anmeldung: **12.09.84 Patentblatt 84/37**

(45) Bekanntmachung des Hinweises auf die Patenterteilung: **12.08.87 Patentblatt 87/33**

(84) Benannte Vertragsstaaten: **AT BE CH DE FR IT LI NL SE**

(56) Entgegenhaltungen:
**EP-A-0 016 729**
**EP-A-0 055 213**
**EP-A-0 065 487**
**EP-A-0 072 438**
**US-A-4 235 777**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Gehret, Jean- Claude, Dr., Im Aeschfeld 12, CH- 4147 Aesch (CH)**
Erfinder: **Gubler, Kurt, Dr., Eulenweg 4, CH- 4125 Riehen (CH)**

**EP 0 118 392 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft substituierte N-Pyrrolylphenyl-N'benzoylharnstoffe, Verfahren zu ihrer Herstellung, Schädlingsbekämpfungsmittel, die sie als aktive Komponente enthalten und ihre Verwendung zur Bekämpfung von Schadinsekten und Vertretern der Ordnung Acarina.

Die erfindungsgemässen N-Pyrrolylphenyl-N'-benzoylharnstoffe haben die Formel I

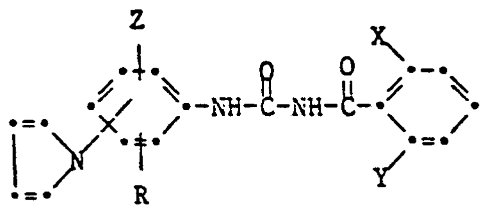

(I)

in welcher

X und Y unabhängig voneinander Wasserstoff oder Halogen,

R Wasserstoff oder Halogen und

Z Wasserstoff, Halogen oder Methyl

bedeuten.

Unter den Verbindungen der Formel I sind diejenigen bevorzugte Wirkstoffe von Schädlingsbekämpfungsmitteln, in denen X und Y unabhängig voneinander Wasserstoff, Fluor oder Chlor, R Wasserstoff oder Chlor und Z Wasserstoff, Chlor oder Methyl bedeuten.

Besonders gute Eigenschaften in dcr Bekämpfung von Schädlingen weisen ferner Verbindungen folgender eingeschränkter Formel Ia auf:

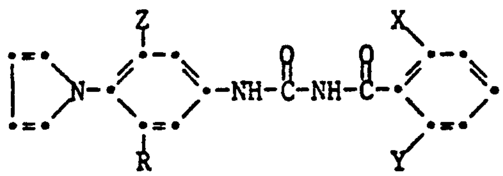

(Ia)

in welcher

X Fluor oder Chlor; Y Wasserstoff oder Fluor und R und Z Chlor bedeuten.

Darüber hinaus sind wegen ihrer hervorragenden Wirksamkeit gegen Schädlinge folgende unter die Formel I fallenden Einzelverbindungen bevorzugte Wirkstoffe:

$N^1$-[3,5-Dichlor-4-pyrrol(1)]-phenyl-$N^2$-2,6-difluorbenzoylharnstoff,

$N^1$-[3,5-Dichlor-4-pyrrol(1)]-phenyl-$N^2$ -2-fluor-6-chlorbenzoylharn-stoff,

$N^1$-[3,5-Dichlor-4-pyrrol(1)]-phenyl-$N^2$-2-chlorbenzoylharnstoff,

$N^1$-(3,5-Dichlor-4-pyrrol(1)]-phenyl-$N^2$-2-fluorbenzoylharnstoff.

Die Verbindungen der Formel I sind neu und nach an sich bekannten Verfahren herstellbar (vgl. u.a. die Deutschen Offenlegungsschriften Nr. 2.123.236, 2.601.780).

So kann eine Verbindung der Formel I beispielsweise erhalten werden durch Umsetzung

a) einer Verbindung der Formel II

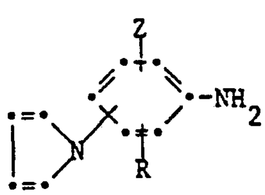

(II)

mit einer Verbindung der Formel III

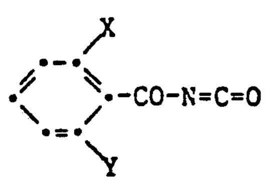

(III)

oder durch Reaktion

b) einer Verbindung der Formel IV

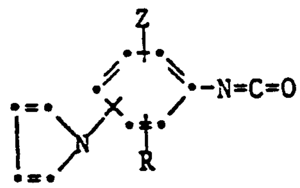

(IV)

mit einer Verbindung der Formel V

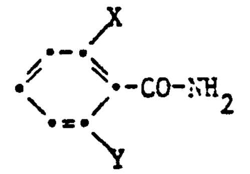

(V).

In den obengenannten Formeln II, III, IV und V haben die Reste R, Z, X und Y die unter Formel I vorstehend angegebenen Bedeutungen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z. B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z. B. Aceton, Methyläthylketon, Methylisopropylketon und Methylisobutylketon. Verfahren a) wird im allgemeinen bei einer Temperatur von -10 bis 100°C, vorzugsweise zwischen 15 und 25°C, gegebenenfalls in Gegenwart einer organischen Base, z. B. Triäthylamin durchgeführt. Die Durchführung von Verfahren b) erfolgt bei einer Temperatur von 0 bis 120°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium.

Die Ausgangsstoffe der vorstehenden Formeln II, III, IV und V sind bekannt oder lassen sich, falls sie neu sind, analog bekannten Verfahren herstellen. So können die Anilinderivate der Formel II durch Reduktion oder katalytische Hydrierung der entsprechenden Nitroverbindungen hergestellt werden [vgl. z. B. Rec. 21, 271 (1902); J. Am. Soc. 68, 1604 (1946); J.Org. Chem. 11, 378 (1946); Rec. 79, 995 (1970)]. Die Isocyanate der Formel IV sind durch Phosgenierung der substituierten Anilinderivate der Formel II nach allgemein üblichen Arbeitsweisen erhältlich. Zu den Verbindungen der Formel III kann man wie folgt gelangen (vgl. J. Agr. Food Chem. 21(3), 348-993; 1973):

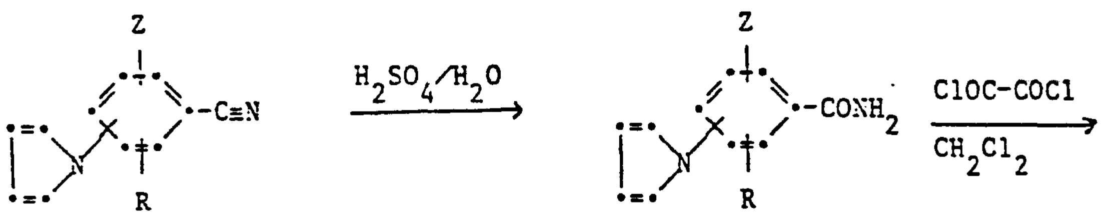


(III)

In den obigen Formeln haben R und Z die unter Formel I angegebenen Bedeutungen.

Die Einführung des Pyrrolringes kann ausgehend von entsprechend substituierten Nitranilinderivaten nach folgendem bekannten Verfahren (vgl. Org. Synthes. 47, 81-82) durchgeführt werden:

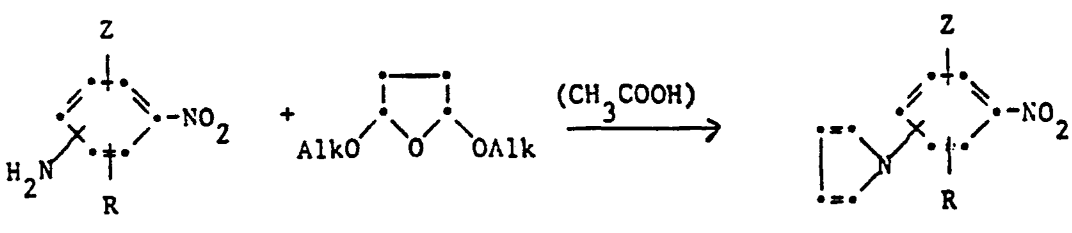


In den obigen Formeln haben R und Z die unter Formel I angegebenen Bedeutungen und Alk stellt Niederalkyl dar.

Die so erhaltenen Nitroverbindungen werden dann, wie oben erwähnt, in die entsprechenden Anilinderivate überführt.

Die neuen Ausgangsverbindungen, welche zu den wertvollen Wirkstoffen der Formel I führen, bilden ebenfalls einen Gegenstand der vorliegenden Erfindung.

Es ist bereits bekannt, dass bestimmte N-Phenyl-N'-benzoylharnstoffe insektizide Eigenschaften besitzen (vgl. deutsche Offenlegungsschriften 2.123.236, 2.504.982, 2.537.413, 2.601.780 und 2.726.684, die belgischen Patentschriften 832.304, 843.906, 844.066 und 867.046 sowie die US-Patentschrift 4.089.975). Aus J. Agr. Food Chem. 21, No. 3, 348ff. (1973) sind weiterhin substituierte N-Phenyl-N'-2,6-dichlorbenzoylharnstoffe bekannt, denen insektizide Eigenschaften zugeschrieben werden. Es hat sich jedoch gezeigt, dass die in den obengenannten Publikationen beschriebenen Benzoylharnstoffe den Erfordernissen der Praxis in der Schädlingsbekämpfung nicht im gewünschten Masse genügen.

Überraschenderweise wurde demgegenüber gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Schädlingen, die Pflanzen und Tiere befallen.

Die Verbindungen der Formel I eignen sich zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Neben der bevorzugten Brauchbarkeit in der Bekämpfung von Fliegen, wie z. B. Musca domestica, und Schmeissfliegen (Blowfly, wie z. B. Lucilia serecata und Lucilia cuprina) und Mücken zeigen die Verbindungen der Formel I auch eine hervorragende Wirkung gegen pflanzenschädigende, Nutz- und Zierpflanzen befallende Frassinsekten. Besonders gut bekämpfbar sind Schadinsekten an Baumwollkulturen, wie z. B. Spodoptera littoralis und Heliothis virescens, und solche an Gemüsekulturen, wie z. B. Leptinotarsa und Pieris brassicae. Vom Wirkungsspektrum der Verbindungen der Formel I ist besonders deren larvizide Wirksamkeit hervorzuheben. Werden z. B. Verbindungen der Formel I von adulten Schadinsekten alimentär aufgenommen, so ist eine starke Reduzierung der Schlupfrate der Larven und Hemmung der Entwicklung der Larvenstadien festzustellen, was eine weitgehende Dezimierung der Populationen der behandelten Schadinsekten zur Folge hat.

Die Verbindungen der Formel I sind ferner einsetzbar zur Bekämpfung von Ektoparasiten an Haus- und Nutztieren, worunter ausser den bereits obengenannten auch solche der Ordnung Acarina zu verstehen sind. Die Bekämpfung der Schädlinge kann mittels Tier-, Stall- und Weidebehandlung durchgeführt werden.

Die erfindungsgemässen Verbindungen der Formel I sind als Wirkstoffe von Mitteln durch die verschiedenen Zubereitungsformen in vielfältiger Weise zur Bekämpfung von Parasiten auf oder in der Umgebung von Tieren, z. B. in Tierstallungen, geeignet. So können sie beispielsweise in Viehbädern (cattle dips), Sprühgängen (spray races), Aufgusslösungen (pouron) oder Handsprühmitteln angewendet werden. Ferner sind sie mit gutem Erfolg für die Behandlung von tierischen Fäkalien mittels der "Feedthrough"-Methode und die stallhygienische Mistbehandlung einsetzbar.

Die Wirkung der erfindungsgemässen Verbindungem bzw. der sie enthaltenden Mittel lässt sich durch Zusatz anderer Insektizide und/oder Akarizide wesentlich verbreitern und an gegebene Umstände anpassen.

Als Zusätze kommen z. B. folgende Wirkstoffe in Betracht:
organische Phosphorverbindungen,
Formamidine, Harnstoffe,
Carbamate,
chlorierte Kohlenwasserstoffe und
Triazinderivate.

Vorteilhafterweise können die Verbindungen der Formel I mit Substanzen kombiniert werden, welche einen biozid verstärkenden Effekt ausüben. Beispiele solcher Verbindungen sind u.a.: Piperonylbutoxid, Propinyläther, Propinyloxime, Propinylcarbamate und Propinylphosphonate oder 2-(3,4-Methylendioxyphenoxy)-3,6,9-trioxaundecan.

Die Verbindungen der Formel I können für sich allein oder zusammen mit geeigneten Trägern und/oder Zusatzstoffen eingesetzt werden. Geeignete Träger und Zusatzstoffe können fest oder flüssig sein und entsprechen den in der Formulierungstechnik üblichen Stoffen, wie z. B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum allgemeinwissen gehören, verarbeitet werden. Ferner sind Viehbäder (cattle dips), Sprühgänge (spray races) und Aufgusslösungen (pour-on-Lösungen), in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen:
Stäubemittel, Streumittel,
Granulate (Umhüllungsgranulate, Imprägnierungsgranulate und Homogengranulate);

Flüssige Aufarbeitungsformen:
a) in Wasser dispergierbare Wirkstoffkonzentrate: Spritzpulver (wettable powders), Pasten, Emulsionen;
b) Lösungen.

Der Gehalt an Wirkstoff in den oben beschriebenen Mitteln liegt zwischen 0,1 bis 95 Gew.-%.

Die Wirkstoffe der Formel I können beispielsweise wie folgt formuliert werden:

Stäubemittel: Zur Herstellung eines a) 5 %igen und b) 2 %igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

Granulat: Zur Herstellung eines 5 %igen Granulates werden die folgenden Stoffe verwendet:
5 Teile Wirkstoff,
0,25 Teile epoxydiertes Pflanzenöl,
0,25 Teile Cetylpolyglykoläther,
3,50 Teile Polyäthylenglykol,
91 Teile Kaolin (Korngrösse 0,3-0,8 mm).

Die Aktivsubstanz wird mit dem epoxydierten Pflanzenöl in 6 Teilen Aceton gelöst, hierauf wird Polyäthylenglykol und Cetylpolyglykoläther zugesetzt. Die so erhaltene Lösung wird auf Koalin aufgesprüht und anschliessend das Aceton im Vakuum verdampft.

Spritzpulver: Zur Herstellung eines a) 40 %igen, b) und c) 25 %iger, d) 10 %igen Spritzpulvers werden folgende Bestandteile verwendet:

a) 40 Teile Wirkstoff,
5 Teile Ligninsulfonsäure-Natriumsslz,
1 Teil Dibutylnaphthalinsulfonsäure-Natriumsalz
54 Teile Kieselsäure;

b) 25 Teile Wirkstoff,
4,5 Teile Calcium-Ligninsulfonat,
1,9 Teile Champagne-Kreide/hydroxyäthylcellulose-Gemisch (1:1),
1,5 Teile Natrium-dibutyl-naphthalinsulfonat,
19,5 Teile Kieselsäure,
19,5 Teile Champagne-Kreide,
28,1 Teile Kaolin;

c) 25 Teile Wirkstoff,
2,5 Teile Isooctylphenoxy-polyoxyäthylen-äthanol,
1,7 Teile Champagne-Kreide/Hydroxyäthylcellulose-Gemisch (1:1)
8,3 Teile Natriumaluminiumsilikat,
16,5 Teile Kieselgur,
46 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zusatzstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10 %igen, b) 25 %igen und c) 50 %igen emulgierbaren Konzentrates werden folgende Stoffe verwendet:

a) 10 Teile Wirkstoff
3,4 Teile epoxydiertes Pflanzenöl,
3,4 Teile eines Kombinationsemulgators, bestehend aus Fett- alkoholpolyglykoläther und Alkylaralkyl-sulfonat-Calcium- Salze,
40 Teile Dimethylformamid,
43,2 Teile Xylol;

b) 25 Teile Wirkstoff
2,5 Teile epoxydiertes Pflanzenöl,
10 Teile eines Alkolarylsulfonat/Fettalkohol-polyglykoläther- Gemisches,
5 Teile Dimethylformamid,
57,5 Teile Xylol;

c) 50 Teile Wirkstoff,
4,2 Teile Tributylphenol-Polyglykoläther,
5,8 Teile Calcium-Dodecylbenzolsulfonat,
20 Teile Cyclohexanon
20 Teile Xylol.

Aus solchen Konzentrationen können durch Verdünnen mit Wasser Emulsionen jeder gewünschten Konzentration hergestellt werden.

Sprühmittel: Zur Herstellung eines a) 5 %igen und b) 95 %igen Sprühmittels werden die folgenden Bestandteile verwendet:

a) 5 Teile Wirkstoff,
1 Teil epoxydiertes Pflanzenöl,
94 Teile Benzin (Siedegrenzen 160-190° C);

b) 95 Teile Wirkstoff,
5 Teile epoxydiertes Pflanzenöl.

**Beispiel 1:**

Herstellung von $N^1$-(3,5-Dichloro-4-pyrrolo(1)]-phenyl-$N^2$-2,6-difluorobenzoylharnstoff

1a) 3,5-Dichloro-4-pyrrolonitrobenzol

Zu 62.1 g (0.3m) 2,6-Dichlor-4-nitroanilin in 500 ml Eisessig werden bei Raumtemperatur 43,6 g (0.33 m) 3,5-Dimethoxytetrahydrofuran gegeben. Die Mischung wird langsam auf 105° erhitzt, 1,5 Stunden bei dieser Temperatur gerührt und anschliessend über Nacht bei Raumtemperatur weitergerürt.

Die dunkle Lösung wird über Hyflo filtriert und am Rotationsverdampfer eingedampft. Die erhaltenen Rohkristalle werden in Eiswasser gut verrührt, filtriert und mit Wasser gewaschen. Nach der Behandlung mit Aktivkohle wird die aus Äthanol gewonnene Substanz umkristallisiert. Ausbeute: 47.7 g (61,8.%) helle Kristalle mit einem Schmelzpunkt von 91-93° C.

1b) 3,5-Dichloro-4-pyrroloanilin

47,7 g (0.18 m) 3,5-Dichloro-4-pyrrolonitrobenzol werden mit 10 g Raney-Nickel bei Raumtemperatur in Tetrahydrofuran hydriert. Nach den Filtrieren wird die bräunliche Lösung am Rotationsverdampfer eingedampft. Die erhaltenen beigen Kristalle werden in Eiswasser aufgeschlämmt, filtriert, mit Hexan gewaschen und im Vakuum bei 40° C getrocknet.

Ausbeute: 39,9 g (94.8 %) helle Kristalle mit einem Schmelzpunkt von 169-171° C.

1c) 2,6-Difluorobenzamid

Zu 240 g konzentrierter Schwefelsäure und 24 ml Wasser bei Raumtemperatur werden 100 g (0.72 m) 2,6-Difluorobenzonitril zugetropft. Die Reaktionsmischung wird 12 Stunden bei 80-85° C gerührt und anschliessend über 1.2 kg Eis/wasser gegossen, 30 min. gerührt und filtriert.

Die Kristalle werden mit Wasser neutral gewaschen und dann bei 85° im Vakuum getrocknet.

Ausbeute: 91.0 g = 80.6 %. Schmelzpunkt: 140-141.5° C.

1d) 2,6-Difluorobenzoylisocyanat

36.1 g (0.23 m) 2,6-Difluorobenzamid werden in 360 ml Methylenchlorid suspendiert. Innerhalb von 20 Min. werden 53.7 g (0.42 m) Oxalylchlorid zugetropft. Nach Beendigung der Salzsäure-Evolution wird über Nacht unter Rückfluss gut gerührt. Das Lösungsmittel wird abdestilliert und der Rückstand über eine 10 cm Vigreuxkolonne bei ca. 80° C /0.3 mm destiliert.

Ausbeute: 37.4 g (89 %) einer leicht gelben Flüssigkeit.

1e) $N^1$-[3,5-Dichloro-4-pyrrolo(1)]-phenyl-$N^2$-2,6-difluorobenzoyl-harnstoff

Zu 10.45 g (0.057 m) 2,6-Difluorobenzoyl-isocyanat in 200 ml Diethylether werden bei Raumtemperatur innerhalb einer Stunde 10.8 g (0.048 m) 3,5-Dichloro-4-pyrroloanilin in 600 ml Diethylether zugetropft. Die Temperatur wird bei 22-24° C gehalten. Nach kurzer Zeit bildet sich ein feiner Niederschlag. Die Reaktionsmischung wird über Nacht weitergerührt und anschliessend filtriert. Der beige Niederschlag wird mit

Hexan gewaschen und bei 40° C im Vakuum getrocknet.
Ausbeute: 15.1 g = 77.4 %, Schmelzpunkt: 235-237° C.
Analoge den vorstehend beschriebenen Arbeitsweisen werden die folgenden Verbindungen der Formel I hergestellt:

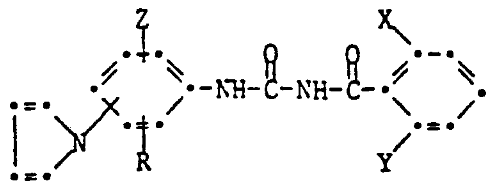

**Tabelle 1**

| Nr | 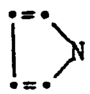 | R | Z | X | Y | Schmelzpunkt [°C] |
|---|---|---|---|---|---|---|
| 1 | 4- | H | H | F | F | 240 - 242 |
| 2 | 4- | 3-Cl | 5-Cl | F | F | 235 - 237 |
| 3 | 4- | H | 5-$CH_3$ | F | F | 204 - 206 |
| 4 | 4- | 3-Cl | 5-Cl | H | Cl | 203 - 204,5 |
| 5 | 4- | 3-Cl | 5-Cl | H | F | 232 - 233 |
| 6 | 4- | 3-Cl | 5-Cl | Cl | Cl | 235 - 237 |
| 7 | 4- | 3-Cl | 5-Cl | F | Cl | 232 - 234 |
| 8 | 2- | H | H | F | F | 179 - 181 |
| 9 | 3- | H | 4-$CH_3$ | F | Cl | 198 - 200 |
| 10 | 2- | H | H | Cl | Cl | 191 - 193 |
| 11 | 2- | H | 5-Cl | F | F | 212 - 214 |

**Beispiel 2**: Wirkung gegen Lucilia Sericata (Blowfly)

1 ml einer wässrigen Suspension bzw. Lösung der Aktivsubstanz mit einem Wirkstoffgehalt von 1000 ppm wurde mit einem speziellen Larvenzuchtmedium bei 50° C so vermischt, dass in einer Verdünnungsreihe homogene Mischungen von 250, 100, 50 und 10 ppm Wirkstoffgehalt hergestellt wurden.
Dann wurden Proben mit ca. 30 frisch geschlüpften Larven (Stadium I) mit den präparierten Larvenzuchtmedien in Kontakt gebracht. Nach 4 Tagen wurde die Mortalitätsrate festgestellt.
Verbindungen gemäss Beispiel 1 und Tabelle 1 zeigten in diesem Test gute Wirksamkeit. So bewirkten die Verbindungen Nr. 2, 4, 5, 6 und 7 bei 10 ppm Wirkstoffkonzentration 100 % Abtötung, während die übrigen Verbindungen bei 250 ppm voll wirksam waren.

**Beispiel 3: Wirkung gegen Lucilia cuprina (Blowfly)**

1 ml einer wässrigen Suspension bzw. Lösung der Aktivsubstanz mit einem Wirkstoffgehalt von 1000 ppm wurde mit einem speziellen Larvenzuchtmedium bei 50° C so vermischt, dass in einer Verdünnungsreihe homogene Mischungen von 250, 100, 50 und 10 ppm Wirkstoffgehalt hergestellt wurden.
Dann wurden Proben mit ca. 30 frisch geschlüpften Larven (Stadium I) mit den präparierten Larvenzuchtmedien in Kontakt gebracht. Nach 4 Tagen wurde die Mortalitätsrate festgestellt.
Verbindungen gemäss Beispiel 1 und Tabelle 1 zeigten in diesem Test gute Wirksamkeit. So bewirkten die Verbindungen Nr. 2, 4, 5, 6 und 7 bei 10 ppm Wirkstoffkonzentration 100 % Abtötung, während die übrigen Verbindungen bei 250 ppm voll wirksam waren.

**Beispiel 4**: Wirkunng gegen Musca domestica

Je 50 frisch zubereitetes CSMA-Nährsubstrat für Maden wurde in Becher eingewogen. Von einer 1 Gew.-%igen acetonischen Lösung des betreffenden Wirkstoffes wurde eine bestimmte Menge auf das in den Bechern befindliche Nährsubstrat pipettiert. Nach dem Durchmischen des Substrates liess man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser vom Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen festgestellt.

Verbindungen gemäss Beispiel 1 und Tabelle 1 bewirkten in diesem Test 100 % Abtötung.

**Beispiel 5**: Wirkung gegen Aedes aegypti

Auf die Oberfläche von 150 ml Wasser, das sich in einem Behälter befindet, wurde so viel einer 0,1 %igen acetonischen Lösung des Wirksteffes pipettiert, dass Konzentrationen von je 10, 5 und 1 ppm erhalten wurden. Nach Verdunsten des Acetons wurde der Behälter mit 30-40 3-tägigen Aëdes-Larven beschickt. Nach 1, 2 und 5 Tagen wurde die Mortalitätsrate ermittelt.

Verbindungen gemäss Beispiel 1 und Tabelle 1 bewirkten in diesem Test bei allen 3 Konzentrationen 100 % Abtötung.

**Beispiel 6**:

Wirkung auf Spodoptera littoralis und Heliothis virescens (Larven und Eier)

Es wurden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages wurden die eingetopften Pflanzen in ein Blechgefäss von etwa 20 Litern Inhalt gestellt, das mit einer Glasplatte abgedeckt wurde. Die Feuchtigkeit im Inneren des abgedeckten Gefässes wurde so reguliert, dass sich kein Kondenswasser bildete. Direktes, auf die Pflanzen fallendes Licht wurde vermieden. Dann wurden die drei Pflanzen infestiert, und zwar insgesamt mit:

a) 50 Larven von Spodoptera littoralis oder Heliothis virescens des ersten larvalen Stadiums;

b) 20 Larven von Spodoptera litteralis oder Heliothis virescens des dritten larvalen Stadiums;

c) zwei Eispiegel von Spodoptera littoralis oder Heliothis virescens. Dazu wurden je 2 Blätter einer Pflanze in einem beidseitig mit Gaze verschlossenen Plexiglaszylinder eingeschlossen; zwei Eispiegel von Spodoptera oder ein Teil eines Baumwollblattes mit darauf abgelegten Eiern von Heliothis wurden zu den eingeschlossenen Blättern gegeben.

Nach 4 bis 5 Tagen erfolgte die Auswertung gegenüber unbehandelten Kontrollen unter Berücksichtigung folgender Kriterien:

a) Anzahl der noch lebenden Larven,

b) Larvale Entwicklungs- und Häutungshemmung,

c) Frasschaden (Schabfrass und Lochfrass),

d) Schlupfrate (Anzahl der aus den Eiern geschlüpften Larven).

Die Verbindungen gemäss Beispiel 1 und Tabelle 1 zeigten in diesem Test gute Gesamt-Wirksamkeit. Die Schlüpfrate betrug weniger als 5 %. Aus den verbliebenen lebenden Larven entwickelten sich keine adulten Organismen.

**Beispiel 7**:

Ovizide Wirkung auf Epilachna varivestis (mexikan. Bohnenkäfer)

Es wurden 20 Gew.-% Wirkstoff, 70 Gew.-% Xylol und 10 Gew.-% einer Mischung aus einem Reaktionsprodukt eines Alkylphenoles mit äthylenoxyd und Calcium-dodecylbenzolsulfonat miteinander vermischt. Aus diesem Konzentrat wurden in einer Verdünnungsreihe wässrige Emulsionen enthaltend 800, 400 und 200 ppm Wirkstoff hergestellt.

Jeweils ca. 100 auf Blätter von Phaseolus vulgaris frisch abgelegte Eier von Epilachna varivestis wurden mit den oben beschriebenen wässrigen Emulsionen (Konzentration 800, 400 bzw. 200 ppm Wirkstoff) angefeuchtet und leicht getrocknet.

In einem belüfteten Gefäss wurden die behandelten Gelege solange gehalten, bis die gleichzeitig angesetzten unbehandelten Kontrollen geschlüpft waren. Unter einem Biocular erfolgte die Auswertung hinsichtlich der erzielten prozentualen Abtötung.

Verbindungen gemäss Beispiel 1 und Tabelle 1 zeigten in diesem Test gute Wirksamkeit. So bewirkten die Verbindungen Nr. 2, 4, 5, 6 und 7 bei 200 ppm Wirkstoffkonzentration 100 % Abtötung, während die übrigen Verbindungen bei 800 ppm voll wirksam waren.

**Beispiel 8**:

Ovizide wirkung auf Heliothis virescens und Leptinotarsa decemlineata

Entsprechende Mengenanteile einer benetzbaren pulverförmigen Formulierung, enthaltend 25 Gew.-% des zu prüfenden Wirkstoffes, wurden mit jeweils soviel Wasser vermischt, dass sich wässrige Emulsionen von ansteigender Wirkstoffkonzentration mit 200, 400 und 800 ppm Wirkstoff ergaben.

In diese wirkstoffhaltigen Emulsionen wurden eintägige Eigelege von Heliothis auf Cellophan bzw. Eingelege von Leptinotarsa auf Kartoffelblättern während drei Minuten eingetaucht und dann auf Rundfiltern abgenutscht. Die so behandelten Gelege wurden in Petrischalen ausgelegt und in der Dunkelheit aufbewahrt. Nach 6 bis 8 Tagen wurde die Schlupfrate im Vergleich zu unbehandelten Kontrollen festgestellt. Zur Auswertung wurde die zur 100 %-igen Abtötung der Eier erforderliche minimale Wirkstoffkonzentration ermittelt.

Verbindungen gemäss Beispiel und Tabelle 1 zeigten in diesem Test gute ovizide Wirkung gegen die geprüften Schädlinge. Die Verbindungen Nr. 2, 4, 5, 6 und 7 bewirkten bei einer Wirkstoffkonzentration von 200 ppm 100 %ige Abtötung, während die übrigen Verbindungen bei 800 ppm voll wirksam waren.

**Beispiel 9**: Wirkung auf Laspetyresia pomonella

Abgelegte Eier von Laspeyresia pomonelle, die nicht älter als 24 Stunden waren, wurden auf Filterpapier für 1 Minute in eine acetonischwässrige Lösung enthaltend 400 ppm des zu prüfenden Wirkstoffes eingetaucht. Nach dem Antrocknen der Lösung wurden die Eier in Petrischalen ausgelegt und bei einer Temperatur von 28°C belassen. Nach 6 Tagen wurde der prozentuale Larvenschlupf aus den behandelten Eiern bewertet. Verbindungen gemäss Beispiel 1 und Tabelle 1 bewirkten in diesem Test keinen Larvenschlupf.

**Beispiel 10**: Chemosterilisierende Wirkung auf Anthonomus grandis

Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, wurden sie zur Kopulation und Eiablage in abgedeckte und Futter enthaltende Schalen eingesetzt. Abgelegte Eier wurden zwei- bis dreimal wöchentlich mit fliessendem Wasser ausgeschwemmt, gezählt, durch zwei- bis dreistündiges Einlagen in ein wässriges Desinfektionsmittel (wie z. B. "Actamer B 100") desinfiziert und dann in Schalen, die eine geeignete Larvaldiät enthielten, deponiert. Nach 7 Tagen wurde erstmals untersucht, ob sich aus den deponierten Eiern Larven entwickelt hatten.

Zur Ermittlung der Dauer des Chemosterilans-Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und geschlüpfter Larven im Vergleich zu unbehandelten Kontrollen.

Verbindungen gemäss Beispiel 1 und Tabelle 1 bewirkten in diesem Test nach 4 Wochen 90 % Reduktion an abgelegten Eiern und 100 % Schlupfunfähigkeit.

**Patentansprüche**

für die Vertragsstaaten: BE, CH, LI, DE, FR, IT, NL, SE

1. Verbindungen der Formel I

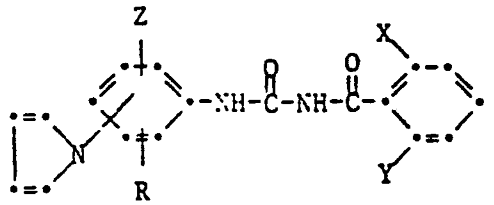

(I)

in welcher
X und Y unabhängig voneinander Wasserstoff oder Halogen,
R Wasserstoff oder Halogen und
Z Wasserstoff, Halogen oder Methyl
bedeuten.

2. Verbindungen gemäss Anspruch 1 der Formel 1a

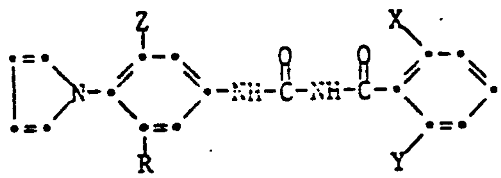

(Ia)

in welcher
X Fluor oder Chlor, Y Wasserstoff oder Fluor und R und Z Chlor bedeuten.

3. Verbindung gemäss Anspruch 2 der Formel N[1]-[3 5-Dichlor-4-pyrrol (1)]-phenyl-N[2]-2,6-difluorbenzoylharnstoff.

4. Verbindung gemäss Anspruch 2 der Formel N[1] -[3,5-Dichlor-4-pyrroll(1)]-phenyl-N[2]-2-fluor-6-chlorbenzoylharnstoff.

5. Verbindung gemäss Anspruch 2 der Formel N[1]-[3,5-Dichlor-4-pyrrol-(1)]phenyl-N[2]-2-chlorbenzoylharnstoff.

6. Verbindung gemäss Anspruch 2 der Formel N[1]-[3,5-Dichlor-4-pyrrol (1)]-phenyl-N[2]-2-fluorbenzoylharnstoff.

7. Verfahren zur Herstellung einer Verbindung gemäss den Ansprüchen 1 bis 6, dadurch gekennzeichnet, dass man

a) eine Verbindung der Formel II

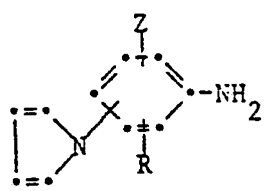

(II)

mit einer Verbindung der Formel III

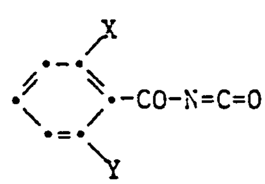

(III)

oder

b) eine Verbindung der Formel IV

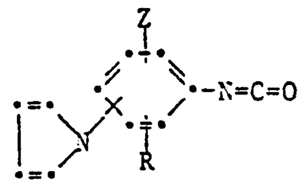

(IV)

mit einer Verbindung der Formel V

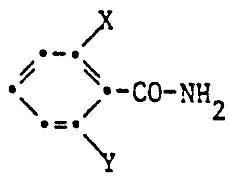

(V)

umsetzt, wobei in den obengenannten Formeln II, III, IV und V die Reste R, Z, X und Y die in den Ansprüchen 1 bis 6 angegebenen Bedeutungen haben.

8. Schädlingsbekämpfungsmittel, welches als aktive Komponente eine Verbindung gemäss den Ansprüchen 1 bis 6 zusammen mit geeigneten Trägern und/oder weiteren Zusatzstoffen enthält.

9. Verbindungen gemäss den Ansprüchen 1 bis 6 zur Anwendung bei der Bekämpfung von Insekten und Vertreter der Ordnung Acarina.

**Patentansprüche**

für den Vertragsstaat: AT

1. Insektizide und akarizide Mittel enthaltend als aktive Komponente mindestens eine Verbindung der Formel I

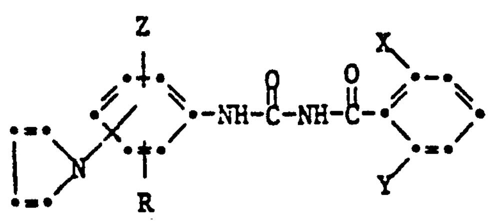

(I)

in welcher
X und Y unabhängig voneinander Wasserstoff oder Halogen,
R Wasserstoff oder Halogen und
Z Wasserstoff, Halogen oder Methyl
bedeuten.

2. Mittel gemäss Anspruch 1, dadurch gekennzeichnet, dass die aktive Komponente eine Verbindung der Formel Ia

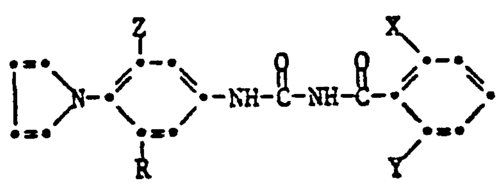

(Ia)

in welcher
X Fluor oder Chlor, Y Wasserstoff oder Fluor und R und Z Chlor bedeuten, ist.

3. Mittel gemäss Anspruch 1, dadurch gekennzeichnet, dass die aktive Komponente die Verbindung $N^1$-[3,5-Dichlor-4-pyrrol(1)]-phenyl-$N^2$-2,6-difluorbenzoylharnstoff ist.

4. Mittel gemäss Anspruch 1, dadurch gekennzeichnet, dass die aktive Komponente die Verbindung $N^1$-[3,5-Dichlor-4-pyrrol(1)]-phenyl-$N^2$-2-fluor-6-chlorbenzoylharnstoff ist.

5. Mittel gemäss Anspruch 1, dadurch gekennzeichnet, dass die aktive Komponente die Verbindung $N^1$-[3,5-Dichlor-4-pyrrol(1)]-phenyl-$N^2$-2-chlorbenzoylharnstoff ist.

6. Mittel gemäss Anspruch 1, dadurch gekennzeichnet, dass die aktive Komponente die Verbindung $N^1$[3,5-Dichlor-4-pyrrol(1)-phenyl-$N^2$-fluorbenzoylharnstoff ist.

7. Verfahren zur Herstellung einer Verbindung der Formel I in Anspruch 1, dadurch gekennzeichnet, dass man

a) eine Verbindung der Formel II

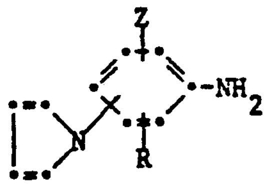

(II)

mit einer Verbindung der Formel III

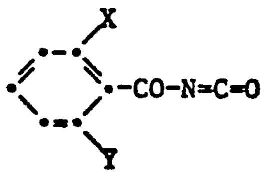

(III)

oder

b) eine Verbindung der Formel IV

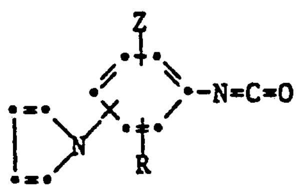

(IV)

mit einer Verbindung der Formel V

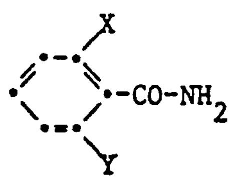

(V)

umsetzt, wobei in den vorgenannten Formeln II, III, IV und V die Reste R, Z, X und Y die in Anspruch 1 angegebenen Bedeutungen haben.

8. Verbindungen der Formel I in Anspruch 1 zur Anwendung bei der Bekämpfung von Insekten und Vertreter der Ordnung Akarina.

**Claims**

the Contracting States: BE, CH, LI, DE, FR, IT, NL, SE

1. A compound of the formula I

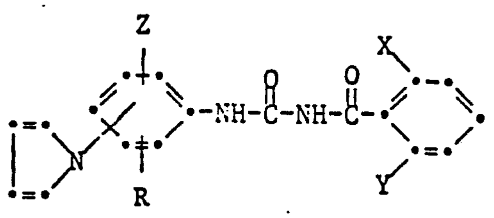

(I)

in which
X and Y are each independently of the other hydrogen or halogen,
R is hydrogen or halogen, and
Z ist hydrogen, halogen or methyl.

2. A compound according to claim 1 of the formula Ia

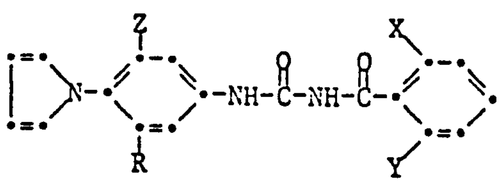

(Ia)

in which X is fluorine or chlorine, Y is hydrogen or fluorine, and R and Z are chlorine.

3. A compound according to claim 2 of the formula $N^1$-[3,5-dichloro-4-pyrrolo(1)]-phenyl-$N^2$-2,6-difluorobenzoylurea.

4. A compound according to claim 2 of the formula $N^1$-[3,5-dichloro-4-pyrrolo(1)]-phenyl-$N^2$-2 -fluoro-6-chlorobenzoylurea.

5. A compound according to claim 2 of the formula $N^1$-[3,5-dichloro-4-pyrrolo(1)]-phenyl-$N^2$-2-chlorobenzoylurea.

6. A compound according to claim 2 of the formula $N^1$-[3 5-dichloro-4-pyrrolo(1)]-phenyl-$N^2$-2-fluorobenzoylurea.

7. A process for the preparation of a compound according to any one of claims 1 to 6, which process comprises

a) reacting a compound of the formula II

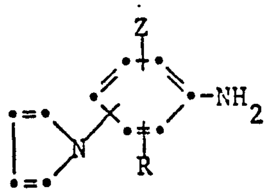

(II)

with a compound of the formula III

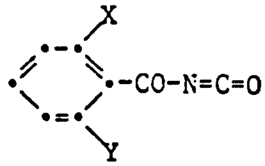

(III); or

b) reacting a compound of the formula IV

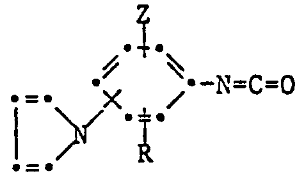

(IV)

with a compound of the formula V

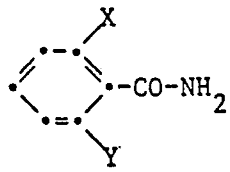

(V),

in which formulae II, III, IV and V the substituents R, Z, X and Y are as defined in claims 1 to 6.

8. A pesticidal composition containing as active ingredient a compound according to any one of claims 1 to 6, together with suitable carriers and/or further adjuvants.

9. A compound as claimed in any one of claims 1 to 6 for use in controlling insects and representatives of the order Acarina.

**Claims**

for the Contracting State: AT

1. An insecticidal and acaricidal composition containing as active component at least one compound of the formula I

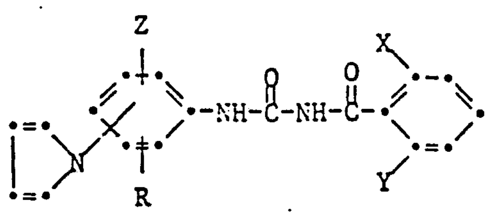

(I)

in which
X and y are each independently of the other hydrogen or halogen,
R is hydrogen or halogen, and
Z is hydrogen, halogen or methyl.

2. A composition according to claim 1 wherein the active component is a compound of the formula Ia

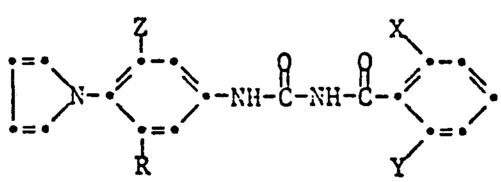

(Ia)

in which X is fluorine or chlorine, Y is hydrogen or fluorine, and R and Z are chlorine.

3. A composition according to claim 1, wherein the active component is N[1]-[3,5-dichloro-4-pyrrolo(1)]-phenyl-N[2]-2,6-difluorobenzoylurea.

4. A composition according to claim 1, wherein the active component is N[1]-[3,5-dichloro-4-pyrrolo(1)]-phenyl-N[2]-2-fluoro-6-chlorobenzoylurea.

5. A composition according to claim 1, wherein the active component is N[1]-[3,5-dichloro-4-pyrrolo(1)]-phenyl-N[2]-2-chlorobenzoylurea.

6. A composition according to claim 1, wherein the active component is N[1]-[3,5-dichloro-4-pyrrolo(l)]-phenyl-N[2]-2-fluorobenzoylurea.

7. A process for the preparation of a compound of formula I according to claim 1, which process comprises

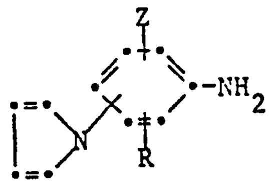

(II)

with a compound of the formula III

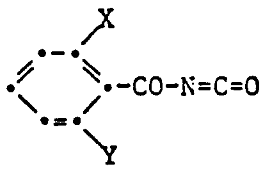

(III); or

b) reacting a compound of the formula IV

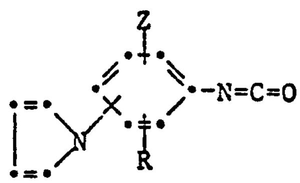

(IV)

with a compound of the formula V

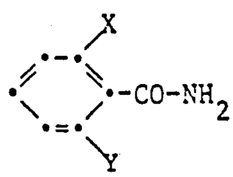

(V),

in which formulae II, III, IV and V the substituents R, Z, X and Y are as defined in claim 1.

8. A compound of formula I according to claim 1 for use in controlling insects and representatives of the order Acarina.

## Revendications

pour les Etats contractants:BE, CH, LI, DE, FR, IT, NL, SE

1. Composés de formule I

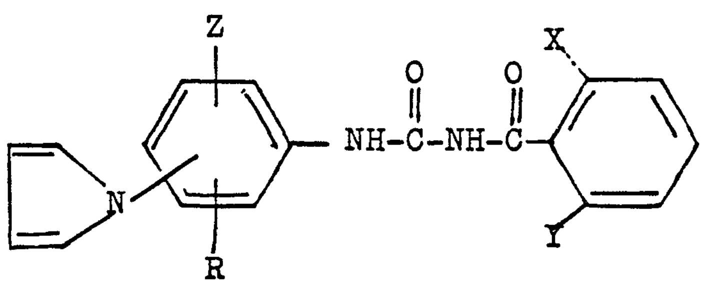

(I)

dans laquelle
X et Y représentent chacun, indépendamment l'un de l'autre, l'hydrogène ou un halogène,
R représente l'hydrogèn ou un halogène, et
Z représente l'hydrogène, un halogène ou un groupe méthyle.

2. Composés selon la revendication 1, répondant à la formule Ia

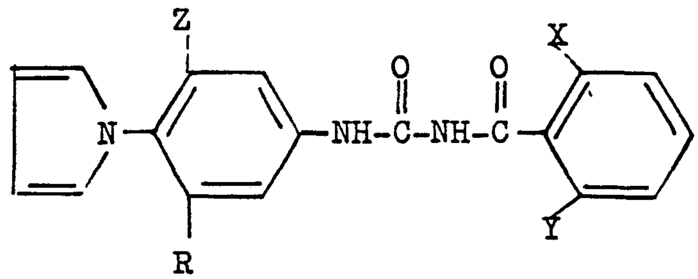

(Ia)

dans laquelle

X représente le fluor ou le chlore, Y l'hydrogène ou le fluor et R et Z le chlore.

3. Composé selon la revendication 2: la $N^1$-[3,5-dichloro-4-pyrrolo-(1)]-phényl-$N^2$-2,6-difluoro-benzoylurée.

4. Composé selon la revendication 2: 1a $N^1$-[3,5-dichloro-4-pyrrolo-(1)]-phényl-$N^2$-2-fluoro-6-chlorobenzoylurée.

5. Composé selon la revendication 2: 1a $N^1$-(3,5-dichloro-4-pyrrolo-(1)]-phényl-$N^2$-2-chloro-benzoylurée.

6. Composé selon la revendication 2: 1a $N^1$-[3,5-dichloro-4-pyrrolo-(1)]-phényl-$N^2$-2-fluoro-benzoylurée.

7 - Procédé de préparation d'un composé selon les revendications 1 à 6, caractérisé en ce que:

a) on fait réagir un composé de formule II

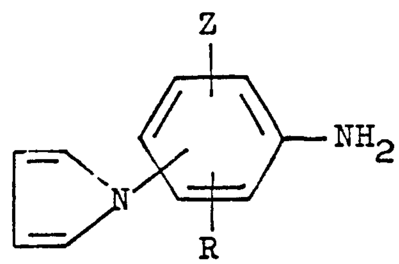

(II)

avec un composé de formule III

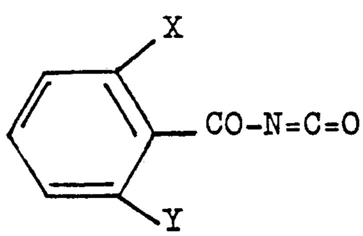

(III)

ou bien

b) on fait réagir un composé de formule IV

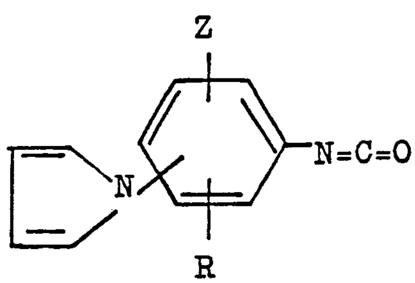

(IV)

avec un composé de formule V

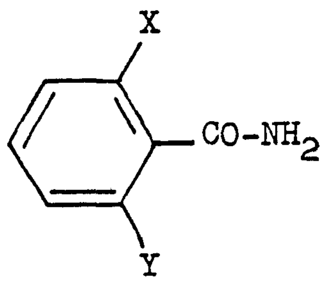

(V)

les symboles R, Z, X et Y ayant dans les formules II, III, IV et V ci-dessus les significations indiquées dans les revendications 1 à 6.

8. Produit pesticide contenant en tant que composant actif un composé selon les revendications 1 à 6 avec des véhicules et/ou d'autres additifs appropriés.

9. Les composés selon les revendications 1 à 6, pour l'utilisation dans la lutte contre les insectes et les représentants de l'ordre des acariens.

**Revendications**

pour l'Etat contractant: AT

1. Produit insecticide et acaricide contenant en tant que composant actif, au moins un composé de formule I

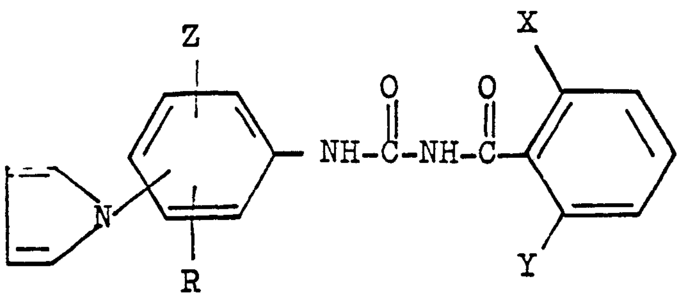

(I)

dans laquelle
X et Y représentent chacun, indépendamment l'un de l'autre, l'hydrogène ou un halogène,
R représente l'hydrogène ou un halogène, et
Z représente l'hydrogène, un halogène ou un groupe méthyle.

2. Produit selon la revendication 1, caractérisé en ce que le composant actif est un composé de formule Ia

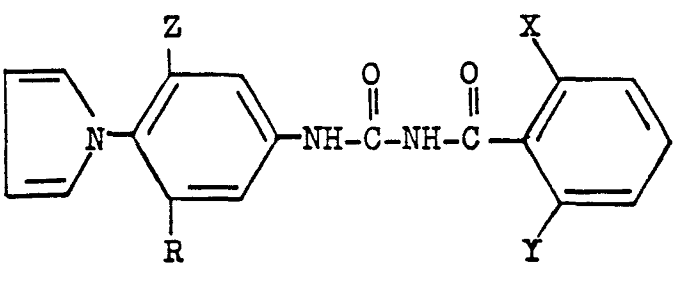

(Ia)

dans laquelle
X représente le fluor ou le chlore, Y l'hgdrogène ou le fluor et R et Z le chlore.

3. Produit selon la revendication 1, caractérisé en ce que le composant actif est la N[1]-[3,5-dichloro-4-pyrrolo-(1)]-phényl-N[2]-2,6-difluorobenzoyl·urée.

4. Produit selon la revendication 1, caractérisé en ce que le composant actif est la N[1]-[3,5-dichloro-4-pyrrolo-(1)]-phényl-N[2]-2-fluoro-6-chloro-benzoylurée.

5. Produit selon la revendication 1, caractérisé en ce que le composant actif est la N[1]-[3,5-dichloro-4-pyrrolo-(1)]-phényl-N[2]-2-chlorobenzoylurée.

6. Produit selon la revendication 1, caractérisé en ce que le composant actif est la N[1]-[3,5-dichloro-4-pyrrolo-

(1)]-phényl-$N^2$-2-fluorobenzoylurée.

7. Procédé de préparation d'un composé de formule 1 de la revendication 1, caractérisé en ce que:

a) on fait réagir un composé de formule II

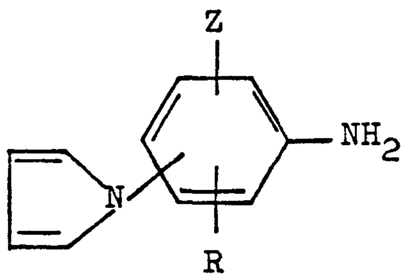

(II)

avec un compose de formule III

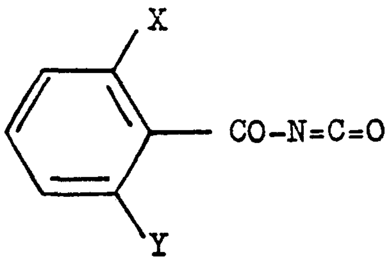

(III)

ou bien

b) on fait réagir un composé de formule IV

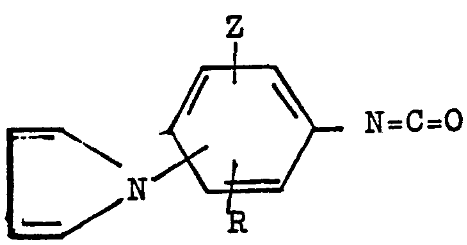

(IV)

avec un composé de formule V

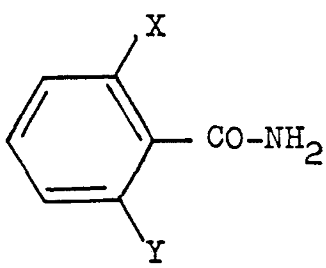

(V)

les symboles R, Z, X et Y ayant dans les formules II, III, IV et V ci-dessus les significations indiquées dans la revendication 1.

8. Composés de formule I de la revendication 1, pour l'utilisation dans la lutte contre les insectes et les représentants de l'ordre des acariens.